# EUROPÄISCHE PATENTSCHRIFT

(11) **EP 2 563 889 B1**
(45) Veröffentlichungstag und Bekanntmachung des Hinweises auf die Patenterteilung: **15.03.2017**
(21) Anmeldenummer: 11721249.8
(22) Anmeldetag: 19.04.2011
(51) Int. Cl.: C11D 1/62, C11D 1/835, C11D 3/00, C11D 3/20, C07C 213/00, C07C 213/06, C07C 219/06

(54) **TEXTILWEICHMACHENDE ZUSAMMENSETZUNG**
TEXTILE SOFTENING COMPOSITION
COMPOSITION D'ADOUCISSANT POUR TEXTILE

(30) Priorität: 28.04.2010 EP 10161296
(43) Veröffentlichungstag der Anmeldung: 06.03.2013
(73) Patentinhaber: Evonik Degussa GmbH, 45128 Essen (DE)
(72) Erfinder: KÖHLE, Hans-Jürgen, 63533 Mainhausen (DE); KOTTKE, Ulrike, 63589 Linsengericht-Großenhausen (DE); WENK, Hans Henning, 45470 Mülheim a. d. Ruhr (DE); JAKOB, Harald, 63594 Hasselroth (DE)
(86) Internationale Anmeldenummer: PCT/EP2011/056185
(87) Internationale Veröffentlichungsnummer: WO 2011/134835

(56) Entgegenhaltungen:
- EP-A2- 0 284 036
- WO-A1-2008/104509
- DE-C1- 19 708 133
- US-A- 5 792 219

## Beschreibung

Gegenstand der Erfindung ist eine textilweichmachende Zusammensetzung, ein Verfahren zur Herstellung einer solchen Zusammensetzung, die Verwendung der Zusammensetzung zur Herstellung eines wässrigen Weichspülers und ein die Zusammensetzung enthaltender wässriger Weichspüler.

Zur Herstellung von wässrigen Weichspülern werden als textilweichmachende Verbindung in der Regel quaternäre Ammoniumsalze eingesetzt, die zwei langkettige Kohlenwasserstoffreste tragen. Mit solchen quaternären Ammoniumsalzen lassen sich stabile wässrige Dispersionen herstellen, die sich als Weichspüler für Textilien eignen und die eine gute weichmachende Wirkung erzielen. Nachteilig ist allerdings die Empfindlichkeit dieser Wirkstoffe gegenüber anionischen Tensiden, deren Anwesenheit die weichmachende Wirkung beeinträchtigt. Es besteht deshalb ein Bedarf an alternativen textilweichmachenden Wirkstoffen, deren weichmachende Wirkung weniger von anionischen Tensiden beeinträchtigt wird.

WO 92/18593 beschreibt partikelförmige textilweichmachende Zusammensetzungen, die einen nichtionischen Weichmacher und ein kationisches Tensid mit nur einem langkettigen C₁₂₋₃₀-Kohlenwasserstoffrest umfassen. Der nichtionische Weichmacher ist nicht substantiv, d.h. er wird aus einer wässrigen Dispersion nicht auf ein Gewebe abgeschieden und kann deshalb für sich allein nicht in einem wässrigen Weichspüler eingesetzt werden. Das kationische Tensid mit nur einem langkettigen C₁₂₋₃₀-Kohlenwasserstoffrest ist zwar substantiv, wirkt aber nicht textilweichmachend. Die Kombination der beiden Komponenten wirkt synergistisch, indem das kationische Tensid den nichtionischen Weichmacher aus wässriger Dispersion auf ein Gewebe aufbringt. Nach der Lehre von WO 92/18593 wird die partikelförmige textilweichmachende Zusammensetzung unmittelbar vor der Anwendung in Wasser dispergiert. Die in den Beispielen von WO 92/18593 beschriebenen textilweichmachenden Zusammensetzungen eignen sich jedoch nicht zur Herstellung von wässrigen Weichspülern, da die aus ihnen erhältlichen Dispersionen nicht ausreichend lagerstabil sind. Darüber hinaus weisen sie auch eine unzureichende weichmachende Wirkung auf.

EP 0 284 036 beschreibt ein Verfahren zur Herstellung von quaternierten Alkanolaminfettsäureestern, bei dem ein Triglycerid mit einem Alkanolamin umgeestert und die erhaltene Reaktionsmischung quaterniert wird. EP 0 284 036 lehrt, zur Herstellung von Monoestern ein Molverhältnis von 1 mol Glycerid zu 3 mol Alkanolamin zu verwenden. Die dabei erhaltenen Zusammensetzungen enthalten ein quaternäres Ammoniumsalz mit einem langkettigen Kohlenwasserstoffrest und einen Glycerinmonofettsäureester in einem molaren Verhältnis von mehr als 2,5:1 und weisen eine unzureichende weichmachende Wirkung auf.

CS 246 532 beschreibt textilweichmachende Zusammensetzungen, die eine synergistische Mischungen aus einem Cholinfettsäureester und einem Fettalkoholethoxylat im Molverhältnis von 0,1:1 bis 8:1 enthalten und von Fettsäuren mit 8 bis 23 Kohlenstoffatomen abgeleitet sind. Die weichmachende Wirkung dieser Zusammensetzungen ist jedoch unbefriedigend.

GB 2 039 556 beschreibt textilweichmachende Zusammensetzungen, die 20 bis 95 mol-% eines kationischen Tensids mit zwei C₁₂₋₂₂-Kohlenwasserstoffresten und 5 bis 80 mol-% einer C₈₋₂₄-Fettsäure umfassen und die zusätzlich bis 50 mol-% eines kationischen Tensids mit nur einem C₁₀₋₂₄-Kohlenwasserstoffrest enthalten können. Nach der Lehre von GB 2 039 556 ist das kationische Tensid mit zwei Kohlenwasserstoffresten eine notwendige Komponente der Zusammensetzung und in den explizit offenbarten Zusammensetzungen ist der molare Anteil an kationischem Tensid mit zwei Kohlenwasserstoffresten stets mindestens so hoch wie der molare Anteil an kationischem Tensid mit einem Kohlenwasserstoffrest.

DE 197 08 133 C1 beschreibt Avivagemittel enthaltend 25 % methylquaternierter Triethanolaminmomotalgfettsäureester-Methylsulfatsalz und 8 % Cetylstearylalkohol.

Die aus dem Stand der Technik bekannten Zusammensetzungen, die einen nichtionischen Weichmacher und ein kationisches Tensid mit nur einem langkettigen Kohlenwasserstoffrest kombinieren, haben im Vergleich zu den üblichen quaternären Ammoniumsalzen mit zwei langkettigen Kohlenwasserstoffresten jedoch alle den Nachteil, dass sie keine vergleichbar hohe weichmachende Wirkung erzielen und meist auch keine ausreichend lagerstabile wässrige Dispersionen liefern.

Es wurde nun überraschend gefunden, dass es zur gleichzeitigen Erzielung einer hohen weichmachenden Wirkung und einer guten Lagerstabilität einer wässrigen Dispersion bei Zusammensetzungen, die einen nichtionischen Weichmacher und ein kationisches Tensid mit nur einem langkettigen Kohlenwasserstoffrest kombinieren, wesentlich darauf ankommt, einen nichtionischen Weichmacher geeigneter Struktur und ein tertiäres oder quaternäres Ammoniumsalz im richtigen molaren Verhältnis zu kombinieren und die Kohlenwasserstoffreste beider Komponenten in der Kettenlänge aufeinander abzustimmen.

Gegenstand der Erfindung ist deshalb eine textilweichmachende Zusammensetzung, die als Komponente A mindestens ein tertiäres oder quaternäres Ammoniumsalz ausgewählt aus Verbindungen der Formeln (I) oder (II):

R¹R²R³N⁺CH₂CHR⁴OC(=O)R⁵ X⁻ (I)

R¹R²R³N⁺(CH₂)₃NHC(=O)R⁵ X⁻ (II)

umfasst, worin
R¹ Wasserstoff, Methyl oder Ethyl ist,
R² und R³ unabhängig voneinander C₁₋₄-Alkyl oder
C₂₋₄-Hydroxyalkyl sind,
R⁴ Wasserstoff oder Methyl ist,
R⁵ ein linearer C₁₅₋₂₁-Alkyl- oder -Alkenylrest ist und
X⁻ ein einwertiges Anion ist
und als Komponente B mindestens einen nichtionischen Weichmacher umfasst, der nur einen langkettigen Kohlenwasserstoffrest mit 15 bis 21 Kohlenstoffatomen gebunden an einen polaren Rest aufweist, wobei der polare Rest maximal 9 Kohlenstoffatome umfasst und mindestens eine freie Hydroxygruppe trägt, umfassend an ein gesättigtes Kohlenstoffatom gebundene alkoholische Hydroxygruppen und an ein Carbonylkohlenstoffatom gebundene carboxylische Hydroxygruppen, und für die
i) das molare Verhältnis der Gesamtmenge an Komponente A zur Gesamtmenge an Komponente B im Bereich von 2:1 bis 1:3 liegt,
ii) der Unterschied zwischen der mittleren Kettenlänge der langkettigen Kohlenwasserstoffreste der Komponenten A und B höchstens 2 Kohlenstoffatome beträgt,
iii) die Kohlenwasserstoffreste der Komponenten A und B im Mittel jeweils höchstens 0,5 Doppelbindungen je Kohlenwasserstoffrest aufweisen,
iv) die Gesamtmenge an tertiären und quaternären Ammoniumsalzen, die zwei langkettige Kohlenwasserstoffrest mit 15 bis 21 Kohlenstoffatomen aufweisen, in der Zusammensetzung höchstens 80 mol-% und vorzugsweise höchstens 50 mol-% der Gesamtmenge an Komponenten A beträgt und
v) der Gesamtanteil an Komponenten A und Komponenten B an der Zusammensetzung mindestens 50 Gew.-% beträgt.

Gegenstand der Erfindung ist außerdem die Verwendung einer erfindungsgemäßen textilweichmachenden Zusammensetzung zur Herstellung eines wässrigen Weichspülers, sowie ein wässriger Weichspüler, der 2 bis 25 Gew.-% einer solchen textilweichmachenden Zusammensetzung und 70 bis 98 Gew.-% Wasser enthält.

Gegenstand der Erfindung sind weiterhin zwei alternative Verfahren zur Herstellung von erfindungsgemäßen textilweichmachenden Zusammensetzungen.

Das erste Verfahren umfasst die Umsetzung einer Mischung enthaltend
n mol einer C₁₆₋₂₂-Fettsäure mit einer Iodzahl von maximal 45,
m mol eines C₂₋₆-Diols oder C₃₋₉-Polyols und
p mol eines Alkanolamins der Formel R²R³NCH₂CHR⁴OH, worin R² und R³ unabhängig voneinander Methyl, 2-Hydroxyethyl oder 2-Hydroxypropyl sind und R⁴ Wasserstoff oder Methyl ist, wobei das Verhältnis von p:m im Bereich von 2:1 bis 1:3 liegt und
n:(p+m) im Bereich von 0,75 bis 1,4 liegt,
bei einer Temperatur im Bereich von 140 bis 210 °C unter Entfernen von Wasser und eine nachfolgende Umsetzung der in Schritt a) erhaltenen Mischung mit einem Alkylierungsmittel bei einer Temperatur im Bereich von 50 bis 100 °C, bis mindestens 90 % der tertiären Aminogruppen quaterniert sind, oder mit einer Säure, bis mindestens 98 % der tertiären Aminogruppen protoniert sind.

Das zweite Verfahren umfasst die Umsetzung einer Mischung enthaltend
n mol einer C₁₆₋₂₂-Fettsäure mit einer Iodzahl von maximal 45,
m mol eines C₂₋₆-Diols oder C₃₋₉-Polyols und
p mol N,N-Dimethyl-1,3-propandiamin,
wobei das Verhältnis von p:m im Bereich von 2:1 bis 1:3 liegt und
n im Bereich von 0,75*m+p bis 1,4*m+p liegt,
bei einer Temperatur im Bereich von 140 bis 210 °C unter Entfernen von Wasser und eine nachfolgende Umsetzung der in Schritt a) erhaltenen Mischung mit einem Alkylierungsmittel bei einer Temperatur im Bereich von 50 bis 100 °C, bis mindestens 90 % der tertiären Aminogruppen quaterniert sind, oder mit einer Säure, bis mindestens 98 % der tertiären Aminogruppen protoniert sind.

Die erfindungsgemäße textilweichmachende Zusammensetzung, umfasst als Komponente A mindestens ein tertiäres oder quaternäres Ammoniumsalz und als Komponente B mindestens einen nichtionischen Weichmacher.

In der erfindungsgemäßen textilweichmachenden Zusammensetzung ist die Komponente A ausgewählt aus Verbindungen der Formeln (I) oder (II):

R¹R²R³N⁺CH₂CHR⁴OC(=O)R⁵ X⁻ (I)

R¹R²R³N⁺(CH₂)₃NHC(=O)R⁵ X⁻ (II)

worin
R¹ Wasserstoff, Methyl oder Ethyl ist,
R² und R³ unabhängig voneinander C₁₋₄-Alkyl oder C₂₋₄-Hydroxyalkyl sind,
R⁴ Wasserstoff oder Methyl ist,
R⁵ ein linearer C₁₅₋₂₁-Alkyl- oder -Alkenylrest ist und
X⁻ ein einwertiges Anion ist.

Die tertiären oder quaternären Ammoniumsalze der Komponente A weisen nur einen langkettigen Kohlenwasserstoffrest auf, der 15 bis 21 Kohlenstoffatome umfasst. Der langkettige Kohlenwasserstoffrest ist vorzugsweise unverzweigt. Tertiäre oder quaternäre Ammoniumsalze, bei denen ein Fettsäurerest über eine Estergruppe oder Amidgruppe und eine verknüpfende Gruppe mit zwei oder drei Kohlenstoffatomen an das Stickstoffatom gebunden ist, weisen gegenüber tertiären oder quaternären Ammoniumsalzen, bei denen ein langkettiger Kohlenwasserstoffrest direkt an das Stickstoffatom des Ammoniumsalzes gebunden ist, eine bessere biologische Abbaubarkeit auf. Vorzugsweise enthält der langkettige Kohlenwasserstoffrest maximal eine Doppelbindung, um eine Oxidation während einer Lagerung zu vermeiden.

Die anderen an das Stickstoffatom des tertiären oder quaternären Ammoniumsalzes gebundenen Gruppen weisen jeweils weniger Kohlenstoffatome auf und sind, neben einem Wasserstoffatom bei einem tertiären Ammoniumsalz, vorzugsweise kurzkettige Gruppen mit jeweils weniger als vier Kohlenstoffatomen und besonders bevorzugt unabhängig voneinander Methyl, Ethyl oder Hydroxyethyl.

In einer bevorzugten Ausführungsform ist in den Formeln (I) und (II) R² ein C₁₋₄-Alkylrest, vorzugsweise ein Methylrest. Besonders bevorzugt sind R² und R³ unabhängig voneinander C₁₋₄-Alkylreste und vorzugsweise Methylreste.

In einer weiteren bevorzugten Ausführungsform sind in Formel (I) R¹ Methyl, R² und R³ unabhängig voneinander Methyl, 2-Hydroxyethyl oder 2-Hydroxypropyl und X⁻ Chlorid oder Methylsulfat und in Formel (II) R¹, R² und R³ Methyl und X⁻ Chlorid oder Methylsulfat. Besonders bevorzugt ist in Formel (I) R² ein Methylrest und am meisten bevorzugt sind in Formel (I) R² und R³ Methylreste.

Das Anion X⁻ ist besonders bevorzugt Methylsulfat.

Beispiele für bevorzugte quaternäre Ammoniumsalze der Komponente A sind Cholinmethylsulfathexadecansäureester, Cholinmethylsulfatoctaadecansäureester, (2-Hydroxypropyl)-trimethylammoniummethylsulfathexadecansäureester, (2-Hydroxypropyl)-trimethylammoniummethylsulfatoctadecansäureester, Bis-(2-hydroxyethyl)-dimethylammoniummethylsulfathexadecansäuremonoester, Bis-(2-hydroxyethyl)-dimethylammoniummethylsulfatoctadecansäuremonoester, Bis-(2-hydroxypropyl)-dimethylammoniummethylsulfathexadecansäuremonoester, Bis-(2-hydroxypropyl)-dimethylammoniummethylsulfatoctadecansäuremonoester, Tris-(2-hydroxyethyl)-methylammoniummethylsulfathexadecansäuremonoester, Tris-(2-hydroxyethyl)-methylammoniummethylsulfatoctadecansäuremonoester, (3-Aminopropyl)-trimethylammoniummethylsulfathexadecansäureamid und (3-Aminopropyl)-trimethylammoniummethylsulfatoctadecansäureamid.

Die erfindungsgemäße textilweichmachende Zusammensetzung, umfasst als Komponente B mindestens einen nichtionischen Weichmacher, der nur einen langkettigen Kohlenwasserstoffrest mit 15 bis 21 Kohlenstoffatomen gebunden an einen polaren Rest aufweist. Der polare Rest umfasst maximal 9 Kohlenstoffatome und trägt mindestens eine freie Hydroxygruppe. Der nichtionische Weichmacher darf jedoch kein Fettalkoholalkoxylat oder Fettsäurealkoxylat sein, da mit der Kombination eines solchen Ethoxylats mit einer Komponente A nur eine unzureichende weichmachende Wirkung erzielt wird. Von der Erfindung ausgenommene Fettalkoholalkoxylate und Fettsäurealkoxylate sind dabei Verbindungen der Formeln (III) und (IV)

R⁵-(OCH₂CHR⁶)ₙ-OH (III)

R⁵-C(=O) (OCH₂CHR⁶)ₙ-OH (IV)

worin
R⁵ ein langkettiger Kohlenwasserstoffrest mit 15 bis 21 Kohlenstoffatomen,
R⁶ Wasserstoff oder ein Alkylrest und
n eine ganze Zahl von 1 bis 4 ist.

Vorzugsweise umfasst der polare Rest maximal 6 Kohlenstoffatome und besonders bevorzugt maximal 3 Kohlenstoffatome. Das Verhältnis von Kohlenstoffatomen zu freien Hydroxygruppen im polaren Rest ist vorzugsweise nicht größer als 3. Freie Hydroxygruppen im Sinne der Erfindung umfassen sowohl alkoholische Hydroxygruppen, die an ein gesättigtes Kohlenstoffatom gebunden sind, als auch carboxylische Hydroxygruppen, die an ein Carbonylkohlenstoffatom gebunden sind.

In einer bevorzugten Ausführungsform ist die Komponente B ausgewählt aus Fettsäuren, Monoestern von Fettsäuren mit einem C₂₋₆-Diol oder C₃₋₉-Polyol und Monoestern von Fettalkoholen mit einer C₂₋₆-Hydroxycarbonsäure. Als C₂₋₆-Diol eignen sich dabei zum Beispiel Ethylenglykol, Diethylenglykol, 1,2-Propandiol, 1,3-Propandiol und Dipropylenglykol. Als C₃₋₉-Polyol eignen sich zum Beispiel Glycerin, Diglycerin, Triglycerin, Sorbit und Sorbitan. Als C₂₋₆-Hydroxycarbonsäure eignen sich zum Beispiel Hdroxyessigsäure, Milchsäure und Äpfelsäure.

Besonders bevorzugt ist die Komponente B ausgewählt aus Fettsäuren und Fettsäuremonoestern von Glycerin, Diglycerin, Triglycerin, Sorbit und Sorbitan.

Beispiele für besonders bevorzugte nichtionische Weichmacher der Komponente B sind Hexadecansäure, Octadecansäure, Glycerinmonohexadecanoat, Glycerinmonooctadecanoat, Diglycerinmonohexadecanoat, Diglycerinmonooctadecanoat, Triglycerinmonohexadecanoat, Triglycerinmonooctadecanoat, Sorbitmonohexadecanoat, Sorbitmonooctadecanoat, Sorbitanmonohexadecanoat und Sorbitanmonooctadecanoat.

Fettsäure im Sinne der Erfindung sind unverzweigte Alkyl-und Alkenylmonocarbonsäuren. Der Begriff Fettsäure umfasst dabei im Sinn der Erfindung nicht nur reine Stoffe, sondern auch Mischungen von Fettsäuren unterschiedlicher Kettenlänge und Mischungen von gesättigten Fettsäuren, wie Palmitinsäure und Stearinsäure, und ungesättigten Fettsäuren, wie Ölsäure und Elaidinsäure. Bevorzugte Fettsäuren sind Mischungen von Alkyl- und Alkenylmonocarbonsäuren aus tierischen und pflanzlichen Quellen, wie zum Beispiel Talgfettsäure, hydrierte Talgfettsäure, Palmfettsäure, hydrierte Rapsfettsäure, hydrierte Sojafettsäure, hydrierte Sonnenblumenfettsäure, sowie Mischungen dieser Fettsäuren.

Die erfindungsgemäße textilweichmachende Zusammensetzung, umfasst die Komponenten A und B in einem molaren Verhältnis der Gesamtmenge an Komponente A zur Gesamtmenge an Komponente B im Bereich von 2:1 bis 1:3 und vorzugsweise im Bereich von 1,5:1 bis 1:1,5. Die Einhaltung des erfindungsgemäßen Verhältnisses der Komponenten A und B ist wesentlich für die Erzielung einer hohen weichmachenden Wirkung.

Der Unterschied zwischen der mittleren Kettenlänge der langkettigen Kohlenwasserstoffreste der Komponenten A und B der erfindungsgemäßen textilweichmachende Zusammensetzung darf höchstens 2 Kohlenstoffatome betragen und beträgt vorzugsweise höchstens 1,5 Kohlenstoffatome. Der Begriff Kettenlänge des langkettigen Kohlenwasserstoffrests bezieht sich dabei nicht auf die Gesamtzahl an Kohlenstoffatomen im Kohlenwasserstoffrest, sondern auf die längste im Kohlenwasserstoffrest enthaltene Kette aus Kohlenstoffatomen. Bei Mischungen von Komponenten A oder Komponenten B mit unterschiedlich langen Kohlenwasserstoffresten berechnet sich die mittlere Kettenlänge aus dem jeweiligen molaren Anteil der einzelnen Verbindungen und ihrer Kettenlänge. Eine Mischung aus 25 mol-% eines quaternären Ammoniumsalzes mit einem linearen C₁₆-Kohlenwasserstoffrest und 75 mol-% eines quaternären Ammoniumsalzes mit einem linearen C₁₈-Kohlenwasserstoffrest weist demnach eine mittlere Kettenlänge von 17,5 auf. Ein Unterschied der mittleren Kettenlänge von mehr als 2 Kohlenstoffatomen führt zu einer verringerten weichmachenden Wirkung und einer schlechteren Lagerstabilität von wässrigen Dispersionen der Zusammensetzung.

Vorzugsweise enthalten die Komponenten A und B den gleichen langkettigen Kohlenwasserstoffrest. Dies kann zum Beispiel dadurch erreicht werden, dass die Komponenten A und B ausgehend von der gleichen Fettsäure oder Fettsäuremischung hergestellt werden.

In der erfindungsgemäßen textilweichmachenden Zusammensetzung dürfen sowohl die Kohlenwasserstoffreste der Komponenten A als auch die Kohlenwasserstoffreste der Komponenten B im Mittel höchstens 0,5 Doppelbindungen je Kohlenwasserstoffrest aufweisen. Bei Mischungen von Komponenten A oder von Komponenten B mit unterschiedlichen Kohlenwasserstoffresten berechnet sich die mittlere Anzahl an Doppelbindungen je Kohlenwasserstoffrest aus dem jeweiligen molaren Anteil der einzelnen Verbindungen. Eine Mischung aus 75 mol-% eines nichtionischen Weichmachers mit einem gesättigten Stearylrest und 25 mol-% eines nichtionischen Weichmachers mit einem einfach ungesättigten Oleylrest weist demnach im Mittel 0,25 Doppelbindungen je Kohlenwasserstoffrest auf. Wenn die Komponenten A oder die Komponenten b mehr als 0,5 Doppelbindungen je Kohlenwasserstoffrest aufweisen, zeigt die Zusammensetzung eine ungenügende weichmachende Wirkung und wässrige Dispersionen der Zusammensetzung weisen eine unzureichende Lagerstabilität auf.

Bei textilweichmachenden Zusammensetzungen, deren Komponenten A oder Komponenten B von einer Fettsäuremischung abgeleitet sind, sind die Komponenten vorzugsweise von einer Fettsäuremischung mit einer Iodzahl von maximal 45 und besonders bevorzugt maximal 25 abgeleitet.

In der erfindungsgemäßen textilweichmachenden Zusammensetzung beträgt die Gesamtmenge an tertiären und quaternären Ammoniumsalzen, die zwei langkettige Kohlenwasserstoffrest mit 15 bis 21 Kohlenstoffatomen aufweisen, höchstens 80 mol-% der Gesamtmenge an Komponenten A, vorzugsweise höchstens 50 mol-% der Gesamtmenge an Komponenten A und besonders bevorzugt höchstens 20 mol-% der Gesamtmenge an Komponenten A. Das molare Verhältnis der Gesamtmenge an tertiären und quaternären Ammoniumsalzen, die nur einen langkettigen Kohlenwasserstoffrest mit 15 bis 21 Kohlenstoffatomen aufweist, zur Gesamtmenge an tertiären und quaternären Ammoniumsalzen, die zwei langkettige Kohlenwasserstoffrest mit 15 bis 21 Kohlenstoffatomen aufweisen, beträgt demnach mindestens 1 : 0,8, vorzugsweise mindestens 1 : 0,5 und besonders bevorzugt mindestens 1 : 0,2. Durch die Beschränkung des Anteils an quaternären Ammoniumsalzen mit zwei langkettigen Kohlenwasserstoffresten lässt sich die weichmachende Wirkung der Zusammensetzung verbessern, insbesondere in Gegenwart von anionischen Tensiden.

Die erfindungsgemäße textilweichmachende Zusammensetzung ist eine konzentrierte Zusammensetzung, bei der der Gesamtanteil an Komponenten A und Komponenten B an der Zusammensetzung mindestens 50 Gew.-% und bevorzugt mindestens 80 Gew.-% beträgt. Die konzentrierten Zusammensetzungen haben den Vorteil, dass sie bei Zugabe zu Wasser oder einer wässrigen Lösung in flüssiger Form selbstdispergierend sind, d. h. sie bilden feinverteilte lagerstabile Dispersionen auch ohne Einwirkung starker Scherkräfte, während bei getrennter Zugaben der Komponenten A und B zu Wasser oder einer wässrigen Lösung hohe Scherkräfte erforderlich sind, um Dispersionen zu erhalten, die sich als Weichspüler einsetzen lassen.

Die konzentrierten erfindungsgemäßen Zusammensetzungen können zusätzlich zu den Komponenten A und B noch ein oder mehrere Lösungsmittel in einer Gesamtmenge von bis zu 20 Gew.-% enthalten. Geeignete Lösungsmittel sind Ethanol, 2-Propanol, Glycerin, Ethylenglykol, 1,2-Propylenglykol, Dipropylenglykol und C1-C4-Alkylmonoether von Ethylenglykol, 1,2-Propylenglykol und Dipropylenglykol, wie zum Beispiel 2-Methoxyethanol, 2-Ethoxyethanol, 2-Butoxyethanol, 1-Methoxy-2-propanol, Dipropylenglykolmonomethylether und Dipropylenglykolmonobutylether. Besonders bevorzugte Lösungsmittel sind Ethanol und 2-Propanol. Ebenfalls als Lösungsmittel geeignet sind Fettsäuretriglyceride mit einer mittleren Kettenlänge des Fettsäurerests von 10 bis 14 Kohlenstoffatomen und einer Iodzahl von 0 bis 15, berechnet für die freie Fettsäure, in einer Gesamtmenge von 2 bis 8 Gew.-% bezogen auf die Gesamtmenge der Zusammensetzung.

Die konzentrierten erfindungsgemäßen Zusammensetzungen enthalten vorzugsweise maximal 10 Gew.-% Wasser und besonders bevorzugt maximal 2 Gew.-% Wasser. Höhere Wassergehalte führen bei den konzentrierten erfindungsgemäßen Zusammensetzungen zu unerwünscht hohen Viskositäten der geschmolzenen Zusammensetzung und können bei Zusammensetzungen, die in den Komponenten A oder B einen über eine Estergruppe gebundenen Fettsäurerest enthalten, zur unerwünschten Hydrolyse der Estergruppe während der Lagerung oder Handhabung der konzentrierten Zusammensetzung führen.

Die erfindungsgemäßen textilweichmachenden Zusammensetzungen lassen sich durch Mischen von mindestens einem tertiären oder quaternären Ammoniumsalz mit den erfindungsgemäßen Eigenschaften und mindestens einem nichtionischen Weichmacher mit den erfindungsgemäßen Eigenschaften im erfindungsgemäßen Molverhältnis herstellen. Geeignete tertiäre oder quaternäre Ammoniumsalze und geeignete nichtionische Weichmacher sind im Handel erhältlich.

Vorzugsweise werden die textilweichmachenden Zusammensetzungen jedoch nach dem erfindungsgemäßen Verfahren hergestellt, bei dem eine Fettsäure, ein C₂₋₆-Diol oder C₃₋₉-Polyol, sowie ein tertiäres Alkanolamin oder ein Diamin mit tertiärer und primärer Aminogruppe in geeignetem Molverhältnis unter Entfernen von Wasser umgesetzt und anschließend alkyliert oder protoniert werden.

In einer ersten Ausführungsform umfasst das erfindungsgemäße Verfahren zur Herstellung der textilweichmachenden Zusammensetzung im ersten Schritt die Umsetzung einer Mischung enthaltend eine C₁₆₋₂₂-Fettsäure mit einer Iodzahl von maximal 45, ein C₂₋₆-Diol oder C₃₋₉-Polyol, sowie ein Alkanolamin der Formel R²R³NCH₂CHR⁴OH, worin R² und R³ unabhängig voneinander Methyl, 2-Hydroxyethyl oder 2-Hydroxypropyl sind und R⁴ Wasserstoff oder Methyl ist. Dabei werden n mol Fettsäure, m mol Diol oder Polyol und p mol Alkanolamin miteinander umgesetzt, wobei das Verhältnis von p zu m im Bereich von 2:1 bis 1:3 liegt und das Verhältnis von n zu (p+m) im Bereich von 0,75 bis 1,4 und vorzugsweise im Bereich von 0,8 bis 1,2 liegt. Die Umsetzung erfolgt bei einer Temperatur im Bereich von 140 bis 210 °C unter Entfernen von Wasser. Das Wasser wird dabei vorzugsweise durch Destillation aus der Mischung entfernt. Während der Umsetzung wird vorzugsweise der Druck von Umgebungsdruck auf einen Druck im Bereich von 5 bis 100 mbar reduziert, um die Entfernung von Wasser zu verstärken. Die Umsetzung kann in Gegenwart eines sauren Katalysators durchgeführt werden, der vorzugsweise in einer Menge von 0,05 bis 0,2 Gew.-% zugesetzt wird. Geeignete saure Katalysatoren sind Methansulfonsäure, p-Toluolsulfonsäure und hypophosphorige Säure. Die Umsetzung im ersten Schritt erfolgt vorzugsweise so lange, bis die Säurezahl der Mischung im Bereich von 1 bis 10 mg KOH/g liegt.

In einem nachfolgenden zweiten Schritt wird die im ersten Schritt erhaltene Mischung entweder mit einem Alkylierungsmittel quaterniert oder mit einer Säure protoniert. Bei der Umsetzung mit einem Alkylierungsmittel erfolgt die Umsetzung bei einer Temperatur im Bereich von 50 bis 100 °C bis mindestens 90 % der tertiären Aminogruppen quaterniert sind. Als Alkylierungsmittel werden bevorzugt Dimethylsulfat oder Methylchlorid verwendet und besonders bevorzugt Dimethylsulfat. Das Alkylierungsmittel wird vorzugsweise in einer Menge von 0,90 bis 0,97 mol und vorzugsweise von 0,92 bis 0,95 mol Alkylierungsmittel je mol eingesetztem Alkanolamin eingesetzt. Bei der Umsetzung mit einer Säure erfolgt die Umsetzung bis mindestens 98 % der tertiären Aminogruppen protoniert sind. Als Säure werden bevorzugt Carbonsäuren mit ein bis drei Kohlenstoffatomen eingesetzt, vorzugsweise Ameisensäure, Essigsäure, Glykolsäure oder Milchsäure und besonders bevorzugt Essigsäure. Die Säure wird dazu vorzugsweise in einer Menge von 0,98 bis 1,2 mol und vorzugsweise von 1,0 bis 1,1 mol Säure je mol eingesetztem Alkanolamin eingesetzt.

In einer zweiten Ausführungsform umfasst das erfindungsgemäße Verfahren zur Herstellung der textilweichmachenden Zusammensetzung im ersten Schritt die Umsetzung einer Mischung enthaltend eine C₁₆₋₂₂-Fettsäure mit einer Iodzahl von maximal 45, ein C₂₋₆-Diol oder C₃₋₉-Polyol, sowie N,N-Dimethyl-1,3-propandiamin. Dabei werden n mol Fettsäure, m mol Diol oder Polyol und p mol Diamin miteinander umgesetzt, wobei das Verhältnis von p zum im Bereich von 2:1 bis 1:3 liegt und n im Bereich von 0,75*m+p bis 1,4*m+p liegt. Die Stoffmenge an Fettsäure wird demnach so gewählt, dass sie der Stoffmenge an Diamin plus dem 0,7-fachen bis 1,4-fachen der Stoffmenge an Diol oder Polyol entspricht. Vorzugsweise liegt n im Bereich von 0,8*m+p bis 1,2*m+p. Die Umsetzung erfolgt im ersten Schritt unter den gleichen Bedingungen wie bei der ersten Ausführungsform des erfindungsgemäßen Verfahrens und kann in gleicher Weise in Gegenwart eines sauren Katalysators durchgeführt werden. Der zweite Schritt des Verfahrens wird bei der zweiten Ausführungsform des erfindungsgemäßen Verfahrens in gleicher Weise durchgeführt wie bei der ersten Ausführungsform des erfindungsgemäßen Verfahrens.

Das erfindungsgemäße Verfahren hat in beiden Ausführungsformen den Vorteil, dass unter Verwendung von nur einem Reaktor beide Komponenten A und B der erfindungsgemäßen Zusammensetzung im erforderlichen Verhältnis aus preiswerten und gut verfügbaren Einsatzstoffen hergestellt werden können. Es macht damit die erfindungsgemäßen Zusammensetzungen in industriellem Maßstab mit geringen Rohstoffkosten und Investitionen zugänglich.

Die mit dem erfindungsgemäßen Verfahren erhältlichen Mischungen sind besonders bevorzugte Ausführungsformen der erfindungsgemäßen Zusammensetzung.

Die erfindungsgemäßen textilweichmachenden Zusammensetzungen und insbesondere die mit dem erfindungsgemäßen Verfahren zugänglichen Zusammensetzungen lassen sich vorteilhaft zur Herstellung eines wässrigen Weichspülers verwenden, da sie lagerstabile wässrige Dispersionen mit multilamellaren Vesikeln bilden und bei Verwendung einer verdünnten Dispersion als Weichspüler eine gute weichmachende Wirkung auf Textilien haben, die vergleichbar hoch ist, wie die weichmachende Wirkung der üblicherweise verwendeten quaternären Ammoniumsalze mit zwei langkettigen Kohlenwasserstoffresten. Überraschenderweise lassen sich durch die Verwendung einer erfindungsgemäßen textilweichmachenden Zusammensetzungen zur Herstellung eines wässrigen Weichspülers im Vergleich zu einer getrennten Zugabe der Komponenten Ammoniumsalz und nichtionischer Weichmacher wässrige Weichspüler mit einer verbesserten weichmachenden Wirkung erhalten.

Die erfindungsgemäßen wässrigen Weichspüler enthalten 2 bis 25 Gew.-% einer erfindungsgemäßen textilweichmachenden Zusammensetzung und 70 bis 98 Gew.-% Wasser. Vorzugsweise enthalten die erfindungsgemäßen Weichspüler eine nach dem erfindungsgemäßen Verfahren erhältliche textilweichmachende Zusammensetzung.

Zusätzlich zu der erfindungsgemäßen textilweichmachenden Zusammensetzung und Wasser können die erfindungsgemäßen Weichspüler weitere Additive und Hilfsstoffe enthalten, insbesondere Parfüm, Farbstoff, Viskositätsregulatoren, Entschäumer, Konservierungsmittel und organische Lösungsmittel.

Als Parfüm können alle für wässrige Weichspüler aus dem Stand der Technik als geeignet bekannten Duftstoffe oder Duftstoffmischungen eingesetzt werden, vorzugsweise in Form eines Parfümöls. Beispiele für geeignete handelsübliche Parfümöle sind Skyline DW 10557 (Hersteller Symrise), White Blossoms DW 10261/7 (Hersteller Symrise), Refresh 154 (Hersteller IFF Inc.) und Passionsblüte PCMf (Hersteller Düllberg Konzentra GmbH). Vorzugsweise wird Parfüm in einer Menge von 0,1 bis 2 Gew.-% verwendet.

Als Farbstoff können alle für wässrige Weichspüler aus dem Stand der Technik als geeignet bekannten Farbstoffe eingesetzt werden, wobei wasserlösliche Farbstoffe bevorzugt sind. Beispiele für geeignete handelsübliche Farbstoffe sind SANDOLAN® Walkblau NBL 150 (Hersteller Clariant), SANDOLAN® Walkgrün N-6GL (Hersteller Clariant) und Sicovit® Azorubin 85 E122 (Hersteller BASF). Vorzugsweise wird Farbstoff in einem Gewichtsanteil von 2 bis 100 ppm verwendet.

Als Viskositätsregulator zur Verringerung der Viskosität kann der wässrige Weichspüler ein Alkalimetall- oder Erdalkalimetallsalz, vorzugsweise Calciumchlorid, in einer Menge von 0,05 bis 2 Gew.-% enthalten. Als Viskositätsregulator zur Erhöhung der Viskosität kann der wässrige Weichspüler einen aus dem Stand der Technik als geeignet bekannten Verdicker enthalten, wobei die aus WO 2007/125005 bekannten Polyurethanverdicker bevorzugt sind. Beispiele für geeignete Verdicker sind TEGO® Visco Plus 3030 (Hersteller Evonik Tego Chemie), Acusol® 880 und 882 (Hersteller Rohm & Haas), Rheovis® CDE (Hersteller BASF), Rohagit® KF 720 F (Hersteller Evonik Röhm GmbH) und Polygel K100 von Neochem GmbH. Viskose Ausführungsformen der erfindungsgemäßen Weichspüler enthalten vorzugsweise einen Verdicker in einer Menge von 0,01 bis 2 Gew.-%.

Als Entschäumer können alle für wässrige Weichspüler aus dem Stand der Technik als geeignet bekannten Entschäumer eingesetzt werden. Beispiele für geeignete handelsübliche Entschäumer sind Dow Corning® DB-110A und TEGO® Antifoam 7001 XP. Vorzugsweise wird Entschäumer in einem Gewichtsanteil von 10 bis 100 ppm verwendet.

Als Konservierungsmittel kann der wässrige Weichspüler aus dem Stand der Technik als geeignet bekannte bakterizide und/oder fungizide Wirkstoffe enthalten, wobei wasserlösliche Wirkstoffe bevorzugt sind. Beispiele für geeignete handelsübliche Bakterizide sind Methylparaben, 2-Brom-2-nitro-1,3-propandiol, 2-methyl-4-isothiazolin-3-on und 5-Chlor-2-methyl-4-isothiazolin-3-on. Ebenso kann der wässrige Weichspüler als Konservierungsmittel einen Oxidationsinhibitor enthalten. Beispiele für geeignete handelsübliche Oxidationsinhibitoren sind Ascorbinsäure, 2,6-Di-tert-butyl-4-methylphenol (BHT), Butylhydroxyanisol (BHA), Tocopherol und Propylgallat. Vorzugsweise werden Bakterizide in einem Gewichtsanteil von 1 bis 2000 ppm und Oxidationsinhibitoren in einem Gewichtsanteil von 10 bis 100 ppm verwendet.

Als organische Lösungsmittel kann der Weichspüler kurzkettige Alkohole, Glykole und Glykolmonoether enthalten, wobei Ethanol, 2-Propanol, 1,2-Propandiol und Dipropylenglykol bevorzugt sind. Vorzugsweise wird organisches Lösungsmittel in einer Menge von 0,2 bis 2 Gew.-% verwendet.

Die erfindungsgemäßen wässrigen Weichspüler werden vorzugsweise durch die Zugabe einer erfindungsgemäßen Zusammensetzung in flüssiger Form zu Wasser oder einer wässrigen Lösung hergestellt. Die Zugabe erfolgt dabei vorzugsweise unter Rühren oder kontinuierlich über einen statischen Mischer. Die erfindungsgemäße Zusammensetzung wird vorzugsweise mit einer Temperatur von höchstens 80 °C, besonders bevorzugt höchstens 75 °C, zugegeben. Die Temperatur des Wassers oder der wässrigen Lösung liegt bei der Zugabe vorzugsweise um 10 bis 15 °C niedriger als die Temperatur der erfindungsgemäßen Zusammensetzung. Die oben genannten Additive und Hilfsstoffe können wahlweise der wässrigen Lösung vor Zugabe der erfindungsgemäßen Zusammensetzung zugefügt werden oder der nach Zugabe der der erfindungsgemäßen Zusammensetzung erhaltenen Dispersion zugefügt werden.

Die Erfindung wird durch die nachfolgenden Beispiele und Vergleichsbeispiele erläutert, die jedoch den Gegenstand der Erfindung nicht beschränken sollen.

### Beispiele

### Herstellung der Zusammensetzungen:

Das Ammoniumsalz und die nichtionische Verbindung wurden, soweit erforderlich, getrennt geschmolzen, entsprechend dem gewünschten molaren Verhältnis flüssig in ein 50 ml Zentrifugenröhrchen aus Polypropylen eingewogen und mit einem Vortexmischer durch Schütteln gemischt.

### Herstellung einer 5 Gew.-% wässrigen Dispersion:

5 Teile der auf 40 bis 80 °C erwärmten flüssigen Zusammensetzung wurden unter Rühren zu 95 Teilen auf 45 bis 65 °C erwärmten Leitungswasser gegeben, 20 min mit einem Propellerrührer bei 45 bis 65 °C gerührt und innerhalb von ca. einer Stunde auf Raumtemperatur abgekühlt.

### Bestimmung der weichmachenden Wirkung:

Die weichmachende Wirkung der Zusammensetzungen wurde in einem sensorischen Test bestimmt, der von einer Gruppe von Testpersonen an Stücken von Baumwollfrotteehandtüchern durchgeführt wurde, die mit einer wässrigen Dispersion der Zusammensetzung behandelt waren. Baumwollfrotteehandtücher von 80 cm auf 50 cm wurden zweimal mit Vollwaschpulver gewaschen, zweimal gespült und geschleudert und auf einer Leine hängend an der Luft getrocknet. Eine wie oben beschrieben hergestellte 5 Gew.-% wässrige Dispersion der Zusammensetzung wurde mit kaltem Leitungswasser zu einer Spüllösung verdünnt, die 0,025 Gew.-% der Zusammensetzung enthielt. Die Baumwollfrotteehandtücher wurden 10 min in 2 l der Spüllösung getaucht, geschleudert und bei Raumtemperatur auf einer Leine hängend an der Luft getrocknet. Danach wurden die behandelten Baumwollfrotteehandtücher in 10 gleiche Stücke von 16 cm auf 25 cm geschnitten und an eine Gruppe von 9 Testpersonen verteilt, die den Weichgriff auf einer Skala von 0 Punkten für hart und unangenehm im Griff bis 5 Punkten für weich und angenehm im Griff bewerteten. Dabei wurden jeweils 2 Zusammensetzungen im Vergleich mit Rewoquat WE18 (Methyltriethanolammoniummethylsulfat-ditalgfettsäureester) und einem unbehandeltem Stück Baumwollfrotteehandtuch beurteilt. Der in den Beispielen angeführte Wert für den Weichgriff ist die Summe der für die Zusammensetzung vergebenen Punkte dividiert durch die Summe der für Rewoquat WE18 vergebenen Punkte, multipliziert mit 100. Werte von mehr als 100 bedeuten demnach einen besseren Weichgriff als mit Rewoquat WE18 und Werte von weniger als 100 einen schlechteren Weichgriff. Wiederholversuche zeigten, dass Unterschiede im Weichgriff von mehr als 10 statistisch signifikant sind.

In den Beispielen bezeichnet Cx jeweils einen Fettsäurerest oder Fettalkylrest mit x Kohlenstoffatomen, wobei C18:1 einen einfach ungesättigten Oleylrest bezeichnet. DMEA-Quat-Cx bezeichnet einen Trimethylethanolammoniummethylsulfat-Fettsäureester einer Fettsäure mit x Kohlenstoffatomen, DMIPA-Quat-Cx den entsprechenden Trimethyl-(2-propanol)-ammoniummethylsulfat-Fettsäureester und DMAPA-Quat-Cx das entsprechende Trimethyl-(3-aminopropyl)-ammoniummethylsulfat-Fettsäureamid. Alle quaternären Ammoniumsalze (DMEA-Quats, DMIPA-Quats, DMAPA-Quats und Rewoquat WE18) enthielten jeweils 15 Gew.-% 2-Propanol.

**Tabelle 1**

| Zusammensetzungen mit Fettsäure als nichtionischer Verbindung | | | | |
|---|---|---|---|---|
| Beispiel | Ammoniumsalz A | Fettsäure B | Molverhältnis A:B | Weichgriff |
| 1* | DMEA-Quat-C18 | C12 | 1:1 | 45 |
| 2* | DMEA-Quat-C18 | C14 | 1:1 | 54 |
| 3 | DMEA-Quat-C18 | C16 | 1:1 | 95 |
| 4 | DMEA-Quat-C18 | C18 | 1:1 | 124 |
| 5* | DMEA-Quat-C18 | C18:1 | 1:1 | 51 |
| 6* | DMEA-Quat-C18:1 | C18:1 | 1:1 | 39 |
| 7 | DMIPA-Quat-C18 | C18 | 1:1 | 94 |
| 8 | DMAPA-Quat-C18 | C18 | 1:1 | 76 |

| | | | | |
|---|---|---|---|---|
| *nicht erfindungsgemäß | | | | |

**Tabelle 2**

| Zusammensetzungen mit Fettalkohol als nichtionischer Verbindung | | | | |
|---|---|---|---|---|
| Beispiel | Ammoniumsalz A | Fettalkohol B | Molverhältnis A:B | Weichgriff |
| 9* | DMEA-Quat-C18 | C12 | 1:1 | 39 |
| 10 | DMEA-Quat-C18 | C18 | 1:1 | 95 |
| 11 | DMEA-Quat-C18 | C18 | 3:2 | 73 |
| 12* | DMEA-Quat-C18 | C18 | 7:3 | 43 |
| 13 | DMAPA-Quat-C18 | C18 | 1:1 | 55 |
| 14* | DMAPA-Quat-C18:1 | C18:1 | 1:1 | 40 |

| | | | | |
|---|---|---|---|---|
| *nicht erfindungsgemäß | | | | |

**Tabelle 3**

| Zusammensetzungen mit Glycerinmonofettsäureester als nichtionischer Verbindung | | | | |
|---|---|---|---|---|
| Beispiel | Ammoniumsalz A | Glycerinmonofettsäureester B | Molverhältnis A:B | Weichgriff |
| 15* | DMEA-Quat-C12 | C12 | 1:1 | 25 |
| 16* | DMEA-Quat-C18 | C12 | 1:1 | 28 |
| 17* | DMEA-Quat-C12 | C18 | 1:1 | 50 |
| 18* | DMEA-Quat-C14 | C18 | 1:1 | 61 |
| 19 | DMEA-Quat-C16 | C18 | 1:1 | 73 |
| 20 | DMEA-Quat-C18 | C18 | 1:1 | 100 |
| 21* | DMEA-Quat-C18 | C18:1 | 1:1 | 51 |
| 22* | DMEA-Quat-C18:1 | C18 | 1:1 | 75 |
| 23* | DMEA-Quat-C18:1 | C18:1 | 1:1 | 51 |
| 24* | DMEA-Quat-C18 | C18 | 1:4 | 75 |
| 25 | DMEA-Quat-C18 | C18 | 3:7 | 88 |
| 26 | DMEA-Quat-C18 | C18 | 2:3 | 88 |
| 27 | DMEA-Quat-C18 | C18 | 3:2 | 94 |
| 28* | DMEA-Quat-C18 | C18 | 7:3 | 72 |
| 29* | DMEA-Quat-C18 | C18 | 4:1 | 58 |
| 30 | DMIPA-Quat-C18 | C18 | 1:1 | 79 |
| 31* | DMAPA-Quat-C12 | C12 | 1:1 | 30 |
| 32* | DMAPA-Quat-C12 | C18 | 1:1 | 61 |
| 33 | DMAPA-Quat-C18 | C18 | 1:1 | 83 |
| 34 | DMEA-HOAc** | C18 | 1:1 | 113 |

| | | | | |
|---|---|---|---|---|
| * nicht erfindungsgemäß ** Dimethylethanolammoniumacetat-Stearinsäureester | | | | |

**Tabelle 4**

| Zusammensetzungen mit Triglycerinmonofettsäureester als nichtionischer Verbindung | | | | |
|---|---|---|---|---|
| Beispiel | Ammoniumsalz A | Triglycerin-monofettsäureester B | Molverhältnis A:B | Weichgriff |
| 35 | DMEA-Quat-C18 | C18 | 1:1 | 81 |
| 36 | DMEA-Quat-C18 | C16/18* | 1:1 | 80 |

| | | | | |
|---|---|---|---|---|
| *Mischung aus Palmitinsäure und Stearinsäure | | | | |

**Tabelle 5**

| Zusammensetzung mit Sorbitanmonofettsäureester als nichtionischer Verbindung | | | | |
|---|---|---|---|---|
| Beispiel | Ammoniumsalz A | Sorbitan-monofettsäureester B | Molverhältnis A:B | Weichgriff |
| 37 | DMEA-Quat-C18 | C16/18* | 1:1 | 89 |

| | | | | |
|---|---|---|---|---|
| *Mischung aus Palmitinsäure und Stearinsäure | | | | |

**Tabelle 6**

| Zusammensetzungen mit Fettsäureethoxylat, Fettalkoholethoxylat oder Fettsäuremethylester als nichtionischer Verbindung | | | | |
|---|---|---|---|---|
| Beispiel | Ammoniumsalz A | Nichtionische Verbindung B | Molverhältnis A:B | Weichgriff |
| 38* | DMEA-Quat-C18 | C18-Fettsäurediethoxylat | 1:1 | 57 |
| 39* | DMAPA-QuatC18:1 | C18:1-Fettsäurediethoxylat | 1:1 | 54 |
| 40* | DMEA-Quat-C18 | C18-Fettalkoholdiethoxylat | 1:1 | 65 |
| 41* | DMEA-Quat-C18 | C18-Fettsäuremethylester | 1:1 | 66 |

| | | | | |
|---|---|---|---|---|
| *nicht erfindungsgemäß | | | | |

### Beispiel 42 (nicht erfindungsgemäß)

Beispiel 4 von EP 0 284 036 A2 wurde nachgearbeitet und das erhaltene Produkt wurde mit ¹H und ¹³C-NMR analysiert. Die erhaltene Zusammensetzung enthielt 58 mol-% Cholinchloridstearinsäureester (Komponente A), 15 mol-% Cholinchlorid, 10 mol-% Glycerin-1-stearat (Komponente B), 5 mol-% Glycerin, 4 mol-% Glycerin-1,3-distearat, 3 mol-% Glycerintristearat, 2 mol-% Glycerin-1,2-distearat und 2 mol-% Stearinsäure (Komponente B). Daraus ergibt sich ein molares Verhältnis der Gesamtmenge an Komponente A zur Gesamtmenge an Komponente B von 58 : 12, entsprechend 4,8 : 1. Für die Zusammensetzung wurde ein Weichgriff von 64 bestimmt.

### Beispiel 43 (nicht erfindungsgemäß)

Beispiel 1 von EP 0 284 036 A2 wurde nachgearbeitet und das erhaltene Produkt wurde mit ¹H und ¹³C-NMR analysiert. Die erhaltene Zusammensetzung enthielt 7,5 mol-% N,N-Dimethyldiethanolammoniumchlorid-Stearinsäurediester, 38,3 mol-% N,N-Dimethyldiethanolammoniumchlorid-Stearinsäuremonoester (Komponente A), 12,0 mol-% N,N-Dimethyldiethanolammoniumchlorid, 5,7 mol-% Glycerintristearat, 15,4 mol-% Glycerindistearat, 14,8 mol % Glycerinmonostearat (Komponente B) und 6,3 mol-% Glycerin. Daraus ergibt sich ein molares Verhältnis der Gesamtmenge an Komponente A zur Gesamtmenge an Komponente B von 2,6. Für die Zusammensetzung wurde ein Weichgriff von 82 bestimmt.

### Beispiel 44 (nicht erfindungsgemäß)

Beispiel 2 von EP 0 284 036 A2 wurde nachgearbeitet und das erhaltene Produkt wurde mit ¹H und ¹³C-NMR analysiert. Die erhaltene Zusammensetzung enthielt 39 mol-% N-Butyl-N-methyl-diethanolammoniumchlorid-Stearinsäuremonoester (Komponente A), 11 mol-% N-Butyl-N-methyldiethanolammoniumchlorid, 11 mol-% N-Butyl-N-methyldiethanolamin-Stearinsäurediester, 17 mol-% N-Butyl-N-methyl-diethanolamin-Stearinsäuremonoester, 6 mol-% Glycerintristearat, 13 mol-% Glycerindistearat, 15 mol % Glycerinmonostearat (Komponente B) und 7 mol-% Glycerin. Daraus ergibt sich ein molares Verhältnis der Gesamtmenge an Komponente A zur Gesamtmenge an Komponente B von 2,6. Für die Zusammensetzung wurde ein Weichgriff von 73 bestimmt.

### Beispiel 45 (nicht erfindungsgemäß)

Beispiel 3 von EP 0 284 036 A2 wurde nachgearbeitet und das erhaltene Umesterungsprodukt wurde mit ¹H und ¹³C-NMR analysiert. Das Umesterungsprodukt enthielt 8,6 mol-% Triethanolamin-Stearinsäurediester, 32,7 mol-% Triethanolamin-Stearinsäuremonoester, 21,8 mol-% Triethanolamin, 10,6 mol-% Glycerintristearat, 8,9 mol-% Glycerindistearat, 8,9 mol % Glycerinmonostearat und 4 mol-% Glycerin. Für den in EP 0 284 036 A2 offenbarten Quaternisierungsgrad von 95 % berechnet sich für das Produkt der Quaternisierung ein Gehalt an N-Methyltriethanolammoniumchlorid-Stearinsäuremonoester von mindestens 29,5 mol-% und damit ein molares Verhältnis der Gesamtmenge an Komponente A zur Gesamtmenge an Komponente B von mehr als 3,3. Der in EP 0 284 036 A2 offenbarte Quaternisierungsgrad konnte bei der Nacharbeitung jedoch nie reproduziert werden. Für die bei der Nacharbeitung erhaltene quaternierte Zusammensetzung wurde ein Weichgriff von 76 bestimmt.

### Beispiel 46

In einem elektrisch beheizten Reaktor mit mechanischem Rührer, Innenthermometer und aufgesetzter Rektifikationskolonne werden 568 g (2,02 mol) Stearinsäure, 108 g (1,21 mol) N,N-Dimethylethanolamin und 93 g (1,01 mol) Glycerin vorgelegt und unter Rühren auf 175 °C erhitzt. Bei dieser Temperatur wird über 8 h unter Rühren Wasser abdestilliert. Die erhaltene Mischung wird auf 60 °C abgekühlt und unter Rühren werden 119,7 g (0,95 mol) Dimethylsulfat so langsam zugegeben, dass die Temperatur nicht über 80 °C steigt. Die erhaltene Mischung wird 1 h bei 80 °C gerührt und mit 129 g 2-Propanol versetzt. Die erhaltene Zusammensetzung wurde mit ¹H und ¹³C-NMR analysiert und enthielt, ohne Berücksichtigung von 2-Propanol, 28 mol-% Cholinmethylsulfatstearinsäureester (Komponente A), 17 mol-% Cholinmethylsulfat, 21 mol-% Glycerin-1-stearat (Komponente B), 8 mol-% Glycerin-2-stearat (Komponente B), 8 mol-% Glycerin-1,3-distearat, 5 mol-% Glycerin-1,2-distearat, 4 mol-% N,N-Dimethylethanolaminstearat, 3 mol-% Stearinsäure (Komponente B), 2 mol-% Glycerintristearat und 2 mol-% Methylstearat. Daraus ergibt sich ein molares Verhältnis der Gesamtmenge an Komponente A zur Gesamtmenge an Komponente B von 28 : 32, entsprechend 1 : 1,1. Für die Zusammensetzung wurde ein Weichgriff von 94 bestimmt.

### Beispiele 47 und 48 (Vergleichsbeispiele)

Beispiel 20 wurde wiederholt, es wurde jedoch keine Mischung aus Cholinmethylsulfatstearinsäureester und Glycerinmonostearat zur Herstellung der 5 Gew.-% wässrigen Dispersion verwendet, sondern es wurde in Beispiel 47 zuerst das Glycerinmonostearat im Wasser dispergiert und erst danach der Cholinmethylsulfatstearinsäureester dispergiert und in Beispiel 48 zuerst der Cholinmethylsulfatstearinsäureester im Wasser dispergiert und erst danach das Glycerinmonostearat dispergiert. Für die Dispersion aus Beispiel 47 wurde ein Weichgriff von 86 und für die Dispersion aus Beispiel 48 ein Weichgriff von 72 bestimmt.

Die Versuche 47 und 48 zeigen, dass durch die Verwendung einer erfindungsgemäßen Zusammensetzung zur Herstellung eines wässrigen Weichspülers gegenüber der Herstellung des Weichspülers aus den einzelnen Komponenten Ammoniumsalz und nichtionischer Weichmacher ein Weichspüler mit verbesserter weichmachender Wirkung erhalten wird.

## Patentansprüche

1. Textilweichmachende Zusammensetzung, umfassend
a) als Komponente A mindestens ein tertiäres oder quaternäres Ammoniumsalz, ausgewählt aus Verbindungen der Formeln (I) oder (II):
R¹R²R³N⁺CH₂CHR⁴OC (=O) R⁵ X⁻ (I)
R¹R²R³N⁺(CH₂) ₃NHC (=O)R⁵ X⁻ (II)
worin
R¹ Wasserstoff, Methyl oder Ethyl ist,
R² und R³ unabhängig voneinander C₁₋₄-Alkyl oder C₂₋₄-Hydroxyalkyl sind,
R⁴ Wasserstoff oder Methyl ist,
R⁵ ein linearer C₁₅₋₂₁-Alkyl- oder -Alkenylrest ist und
X⁻ ein einwertiges Anion ist, und
b) als Komponente B mindestens einen nichtionischen Weichmacher, der nur einen langkettigen Kohlenwasserstoffrest mit 15 bis 21 Kohlenstoffatomen gebunden an einen polaren Rest aufweist, wobei der polare Rest maximal 9 Kohlenstoffatome umfasst und mindestens eine freie Hydroxygruppe trägt, umfassend an ein gesättigtes Kohlenstoffatom gebundene alkoholische Hydroxygruppen und an ein Carbonylkohlenstoffatom gebundene carboxylische Hydroxygruppen, und wobei der nichtionische Weichmacher kein Fettalkoholalkoxylat oder Fettsäurealkoxylat ist,
wobei
i) das molare Verhältnis der Gesamtmenge an Komponente A zur Gesamtmenge an Komponente B im Bereich von 2:1 bis 1:3 liegt,
ii) der Unterschied zwischen der mittleren Kettenlänge der langkettigen Kohlenwasserstoffreste der Komponenten A und B höchstens 2 Kohlenstoffatome beträgt,
iii) die Kohlenwasserstoffreste der Komponenten A und B im Mittel jeweils höchstens 0,5 Doppelbindungen je Kohlenwasserstoffrest aufweisen,
iv) die Gesamtmenge an tertiären und quaternären Ammoniumsalzen, die zwei langkettige Kohlenwasserstoffrest mit 15 bis 21 Kohlenstoffatomen aufweisen, in der Zusammensetzung höchstens 80 mol-% und vorzugsweise höchstens 50 mol-% der Gesamtmenge an Komponenten A beträgt und
v) der Gesamtanteil an Komponenten A und Komponenten B an der Zusammensetzung mindestens 50 Gew.-% beträgt.

2. Textilweichmachende Zusammensetzung nach Anspruch 1, **dadurch gekennzeichnet, dass** der Gesamtanteil an Komponenten A und Komponenten B an der Zusammensetzung mindestens 80 Gew.-% beträgt.

3. Textilweichmachende Zusammensetzung nach Anspruch 1 oder 2, **dadurch gekennzeichnet, dass** die Zusammensetzung maximal 10 Gew.-% Wasser enthält.

4. Textilweichmachende Zusammensetzung nach einem der Ansprüche 1 bis 3, **dadurch gekennzeichnet, dass** die Komponente B ausgewählt ist aus der Gruppe bestehend aus Fettsäuren, Monoestern von Fettsäuren mit einem C₂₋₆-Diol oder C₃₋₉-Polyol, und Monoestern von Fettalkoholen mit einer C₂₋₆-Hydroxycarbonsäure.

5. Textilweichmachende Zusammensetzung nach Anspruch 4, **dadurch gekennzeichnet, dass** die Komponente B ausgewählt ist aus der Gruppe bestehend aus Fettsäuren und Fettsäuremonoestern von Glycerin, Diglycerin, Triglycerin, Sorbit und Sorbitan.

6. Textilweichmachende Zusammensetzung nach einem der Ansprüche 1 bis 5, **dadurch gekennzeichnet, dass** in den Formeln (I) und (II) R² ein C₁₋₄-Alkylrest und vorzugsweise ein Methylrest ist.

7. Textilweichmachende Zusammensetzung nach einem der Ansprüche 1 bis 6, **dadurch gekennzeichnet, dass** in den Formeln (I) und (II) R² und R³ unabhängig voneinander C₁₋₄-Alkylreste und vorzugsweise Methylreste sind.

8. Textilweichmachende Zusammensetzung nach einem der Ansprüche 1 bis 7, **dadurch gekennzeichnet, dass** R¹ Methyl ist, in Formel (I) R² und R³ unabhängig voneinander Methyl, 2-Hydroxyethyl oder 2-Hydroxypropyl und in Formel (II) R² und R³ Methyl sind und X⁻ Chlorid oder Methylsulfat ist.

9. Textilweichmachende Zusammensetzung nach einem der Ansprüche 1 bis 8, **dadurch gekennzeichnet, dass** Komponente A und Komponente B den gleichen langkettigen Kohlenwasserstoffrest enthalten.

10. Verfahren zur Herstellung einer textilweichmachenden Zusammensetzung, umfassend
a) Umsetzung einer Mischung enthaltend
n mol einer C₁₆₋₂₂-Fettsäure mit einer Iodzahl von maximal 45,
m mol eines C₂₋₆-Diols oder C₃₋₉-Polyols und
p mol eines Alkanolamins der Formel R²R³NCH₂CHR⁴OH, worin R² und R³ unabhängig voneinander Methyl, 2-Hydroxyethyl oder 2-Hydroxypropyl sind und R⁴ Wasserstoff oder Methyl ist,
wobei das Verhältnis von p:m im Bereich von 2:1 bis 1:3 liegt und
n:(p+m) im Bereich von 0,75 bis 1,4 liegt,
bei einer Temperatur im Bereich von 140 bis 210 °C unter Entfernen von Wasser und
b) Umsetzung der in Schritt a) erhaltenen Mischung mit einem Alkylierungsmittel bei einer Temperatur im Bereich von 50 bis 100 °C, bis mindestens 90 % der tertiären Aminogruppen quaterniert sind, oder mit einer Säure, bis mindestens 98 % der tertiären Aminogruppen protoniert sind.

11. Verfahren zur Herstellung einer textilweichmachenden Zusammensetzung, umfassend
a) Umsetzung einer Mischung enthaltend
n mol einer C₁₆₋₂₂-Fettsäure mit einer Iodzahl von maximal 45,
m mol eines C₂₋₆-Diols oder C₃₋₉-Polyols und
p mol N,N-Dimethyl-1,3-propandiamin,
wobei das Verhältnis von p:m im Bereich von 2:1 bis 1:3 liegt und
n im Bereich von 0,75*m+p bis 1,4*m+p liegt,
bei einer Temperatur im Bereich von 140 bis 210 °C unter Entfernen von Wasser und
b) Umsetzung der in Schritt a) erhaltenen Mischung mit einem Alkylierungsmittel bei einer Temperatur im Bereich von 50 bis 100 °C, bis mindestens 90 % der tertiären Aminogruppen quaterniert sind, oder mit einer Säure, bis mindestens 98 % der tertiären Aminogruppen protoniert sind.

12. Verfahren nach Anspruch 10 oder 11, **dadurch gekennzeichnet, dass** das Alkylierungsmittel Dimethylsulfat oder Methylchlorid ist.

13. Verfahren nach Anspruch 10 oder 11, **dadurch gekennzeichnet, dass** die Säure eine Carbonsäure mit 1 bis 3 Kohlenstoffatomen ist.

14. Textilweichmachende Zusammensetzung erhältlich nach einem Verfahren gemäß einem der Ansprüche 10 bis 13.

15. Wässriger Weichspüler, enthaltend 2 bis 25 Gew.-% einer textilweichmachenden Zusammensetzung gemäß einem der Ansprüche 1 bis 9 oder gemäß Anspruch 14 und 70 bis 98 Gew.-% Wasser.

16. Verwendung einer textilweichmachenden Zusammensetzung gemäß einem der Ansprüche 1 bis 9 oder gemäß Anspruch 14 zur Herstellung eines wässrigen Weichspülers.

## Claims

1. Fabric softening composition comprising
a) as component A at least one tertiary or quaternary ammonium salt, selected from compounds of the formulae (I) and (II):
R¹R²R³N⁺CH₂CHR⁴OC(=O) R⁵ X⁻ (I)
R¹R²R³N⁺(CH₂)₃NHC(=O)R⁵ X- (II)
in which
R¹ is hydrogen, methyl or ethyl,
R² and R³, independently of one another, are C₁₋₄ alkyl or C₂₋₄ hydroxyalkyl,
R⁴ is hydrogen or methyl,
R⁵ is a linear C₁₅₋₂₁ alkyl or alkenyl radical and
X⁻ is a monovalent anion,
and
b) as component B at least one nonionic softener which has only one long-chain hydrocarbon radical having 15 to 21 carbon atoms bonded to a polar radical, where the polar radical comprises at most 9 carbon atoms and carries at least one free hydroxy group, comprising alcoholic hydroxy groups bonded to a saturated carbon atom and carboxylic hydroxy groups bonded to a carbonyl carbon atom, and where the nonionic softener is not a fatty alcohol alkoxylate or fatty acid alkoxylate,
where
i) the molar ratio of the total amount of component A to the total amount of component B is in the range from 2:1 to 1:3,
ii) the difference between the average chain length of the long-chain hydrocarbon radicals of components A and B is at most 2 carbon atoms,
iii) the hydrocarbon radicals of components A and B have on average in each case at most 0.5 double bonds per hydrocarbon radical,
iv) the total amount of tertiary and quaternary ammonium salts, carrying two long-chain hydrocarbon radicals of 15 to 21 carbon atoms, in the composition is at most 80 mol% and preferably at most 50 mol% of the total amount of components A and
v) the total fraction of components A and components B in the composition is at least 50% by weight.

2. Fabric softening composition according to Claim 1, **characterized in that** the total fraction of components A and components B in the composition is at least 80% by weight.

3. Fabric softening composition according to Claim 1 or 2, **characterized in that** the composition comprises at most 10% by weight of water.

4. Fabric softening composition according to any one of Claims 1 to 3, **characterized in that** component B is selected from the group consisting of fatty acids, monoesters of fatty acids with a C₂₋₆ diol or
C₃₋₉ polyol, and monoesters of fatty alcohols with a C₂₋₆ hydroxycarboxylic acid.

5. Fabric softening composition according to Claim 4, **characterized in that** component B is selected from the group consisting of fatty acids and fatty acid monoesters of glycerol, diglycerol, triglycerol, sorbitol and sorbitan.

6. Fabric softening composition according to any one of Claims 1 to 5, **characterized in that** in the formulae (I) and (II) R² is a C₁₋₄ alkyl radial and preferably a methyl radical.

7. Fabric softening composition according to any one of Claims 1 to 6, **characterized in that** in the formulae (I) and (II) R² and R³, independently of one another, are C₁₋₄ alkyl radicals and preferably methyl radicals.

8. Fabric softening composition according to any one of Claims 1 to 7, **characterized in that** R¹ is methyl, in formula (I) R² and R³, independently of one another, are methyl, 2-hydroxyethyl or 2-hydroxypropyl, in formula (II) R² and R³ are methyl, and X⁻ is chloride or methylsulphate.

9. Fabric softening composition according to any one of Claims 1 to 8, **characterized in that** component A and component B comprise the same long-chain hydrocarbon radical.

10. Process for producing a fabric softening composition, comprising
a) reacting a mixture comprising
n mol of a C₁₆₋₂₂ fatty acid with an iodine number of at most 45,
m mol of a C₂₋₆ diol or C₃₋₉ polyol and
p mol of an alkanolamine of the formula R²R³NCH₂CHR⁴OH, in which R² and R³, independently of one another, are methyl, 2-hydroxyethyl or 2-hydroxypropyl and R⁴ is hydrogen or methyl, where the ratio of p:m is in the range from 2:1 to 1:3 and
n:(p+m) is in the range from 0.75 to 1.4,
at a temperature in the range from 140 to 210°C with removal of water and
b) reacting the mixture obtained in step a) with an alkylating agent at a temperature in the range from 50 to 100°C until at least 90% of the tertiary amino groups have been quaternized, or with an acid until at least 98% of the tertiary amino groups have been protonated.

11. Process for producing a fabric softening composition, comprising
a) reacting a mixture comprising
n mol of a C₁₆₋₂₂ fatty acid with an iodine number of at most 45,
m mol of a C₂₋₆ diol or C₃₋₉ polyol and
p mol of N,N-dimethyl-1,3-propanediamine, where the ratio of p:m is in the range from 2:1 to 1:3 and
n is in the range from 0.75*m+p to 1.4*m+p,
at a temperature in the range from 140 to 210°C with removal of water and
b) reacting the mixture obtained in step a) with an alkylating agent at a temperature in the range from 50 to 100°C until at least 90% of the tertiary amino groups have been quaternized, or with an acid until at least 98% of the tertiary amino groups have been protonated.

12. Process according to Claim 10 or 11, **characterized in that** the alkylating agent is dimethyl sulphate or methyl chloride.

13. Process according to Claim 10 or 11, **characterized in that** the acid is a carboxylic acid having 1 to 3 carbon atoms.

14. Fabric softening composition obtainable by a process according to any one of Claims 10 to 13.

15. Aqueous rinse cycle fabric softener comprising
2 to 25% by weight of a fabric softening composition according to any one of Claims 1 to 9 or according to Claim 14 and
70 to 98% by weight of water.

16. Use of a fabric softening composition according to any one of Claims 1 to 9 or according to Claim 14 for producing an aqueous rinse cycle fabric softener.

## Revendications

1. Composition d'adoucissement de textiles, comprenant :
a) comme composant A au moins un sel d'ammonium tertiaire ou quaternaire, choisi parmi les composés des formules (I) ou (II) :
R¹R²R³N⁺CH₂CHR⁴OC (=O) R⁵X⁻ (I)
R¹R²R³N⁺ (CH₂) ₃NHC (=O) R⁵X⁻ (II)
dans lesquelles
R¹ représente hydrogène, méthyle ou éthyle,
R² et R³ représentent, indépendamment l'un de l'autre, C₁₋₄-alkyle et C₂₋₄-hydroxyalkyle,
R⁴ représente hydrogène ou méthyle,
R⁵ représente un radical C₁₅₋₂₁-alkyle ou C₁₅₋₂₁-alcényle linéaire et
X⁻ représente un anion monovalent et
b) comme composant B au moins un assouplissant non ionique, qui présente uniquement un radical hydrocarboné à longue chaîne comprenant 15 à 21 atomes de carbone lié à un radical polaire, le radical polaire comprenant au maximum 9 atomes de carbone et portant au moins un groupe hydroxy libre, comprenant des groupes hydroxy alcoolique liés à un atome de carbone saturé et des groupes hydroxy carboxylique liés à un atome de carbone à fonction carbonyle, et l'adoucissant non ionique n'étant pas un alcoxylate d'alcool gras ni un alcoxylate d'acide gras,
i) le rapport molaire de la quantité totale de composant A à la quantité totale de composant B se situant dans la plage de 2:1 à 1:3,
ii) la différence entre la longueur moyenne de chaîne des radicaux hydrocarbonés à longue chaîne des composants A et B étant d'au plus 2 atomes de carbone,
iii) les radicaux hydrocarbonés des composants A et B présentant en moyenne à chaque fois au plus 0,5 double liaison par radical hydrocarboné,
iv) la quantité totale de sels d'ammonium tertiaire et quaternaire, qui présentent deux radicaux hydrocarbonés à longue chaîne comprenant 15 à 21 atomes de carbone, représentant, dans la composition, au plus 80% en mole et de préférence au plus 50% en mole de la quantité totale de composant A et
v) la quantité totale de composant A et de composant B, par rapport à la composition, représentant au moins 50% en poids.

2. Composition d'adoucissement de textiles selon la revendication 1, **caractérisée en ce que** la quantité totale de composant A et de composant B, par rapport à la composition, représentant au moins 80% en poids.

3. Composition d'adoucissement de textiles selon la revendication 1 ou 2, **caractérisée en ce que** la composition contient au maximum 10% en poids d'eau.

4. Composition d'adoucissement de textiles selon l'une quelconque des revendications 1 à 3, **caractérisée en ce que** le composant B est choisi dans le groupe constitué par les acides gras, les monoesters d'acides gras avec un C₂₋₆-diol ou un C₃₋₉-polyol et les monoesters d'alcools gras avec un acide C₂₋₆-hydroxycarboxlique.

5. Composition d'adoucissement de textiles selon la revendication 4, **caractérisée en ce que** le composant B est choisi dans le groupe constitué par les acides gras et les monoesters d'acides gras du glycérol, du diglycérol, du triglycérol, du sorbitol et du sorbitane.

6. Composition d'adoucissement de textiles selon l'une quelconque des revendications 1 à 5, **caractérisée en ce que**, dans les formules (I) et (II), R² représente un radical C₁₋₄-alkyle et de préférence un radical méthyle.

7. Composition d'adoucissement de textiles selon l'une quelconque des revendications 1 à 6, **caractérisée en ce que**, dans les formules (I) et (II), R² et R³ représentent, indépendamment l'un de l'autre, des radicaux C₁₋₄-alkyle et de préférence des radicaux méthyle.

8. Composition d'adoucissement de textiles selon l'une quelconque des revendications 1 à 7, **caractérisée en ce que** R¹ représente méthyle, dans la formule (I), R² et R³ représentent, indépendamment l'un de l'autre, méthyle, 2-hydroxyéthyle ou 2-hydroxypropyle et, dans la formule (II), R² et R³ représentent méthyle ; et X⁻ représente chlorure ou méthylsulfate.

9. Composition d'adoucissement de textiles selon l'une quelconque des revendications 1 à 8, **caractérisée en ce que** le composant A et le composant B contiennent le même radical hydrocarboné à longue chaîne.

10. Procédé pour la préparation d'une composition d'adoucissement de textiles, comprenant
a) réaction d'un mélange contenant
n moles d'un acide gras en C₁₆₋₂₂ présentant un indice d'iode d'au maximum 45,
m moles d'un C₂₋₆-diol ou d'un C₃₋₉-polyol et
p moles d'une alcanolamine de formule R²R³NCH₂CHR⁴OH,
dans laquelle R² et R³ représentent, indépendamment l'un de l'autre, méthyle, 2-hydroxyéthyle ou 2-hydroxypropyle et R⁴ représente hydrogène ou méthyle,
le rapport p:m se situant dans la plage de 2:1 à 1:3 et
n:(p+m) se situant dans la plage de 0,75 à 1,4,
à une température dans la plage de 140 à 210°C avec élimination d'eau et
b) réaction du mélange obtenu dans l'étape a) avec un agent d'alkylation à une température dans la plage de 50 à 100°C jusqu'à ce qu'au moins 90% des groupes amino tertiaire soient quaternisés ou avec un acide jusqu'à ce qu'au moins 98% des groupes amino tertiaire soient protonés.

11. Procédé pour la préparation d'une composition d'adoucissement de textiles, comprenant
a) réaction d'un mélange contenant
n moles d'un acide gras en C₁₆₋₂₂ présentant un indice d'iode d'au maximum 45,
m moles d'un C₂₋₆-diol ou d'un C₃₋₉-polyol et
p moles de N,N-diméthyl-1,3-propanediamine,
le rapport p:m se situant dans la plage de 2:1 à 1:3 et
n se situant dans la plage de 0,75*m+p à 1,4*m+p,
à une température dans la plage de 140 à 210°C avec élimination d'eau et
b) réaction du mélange obtenu dans l'étape a) avec un agent d'alkylation à une température dans la plage de 50 à 100°C jusqu'à ce qu'au moins 90% des groupes amino tertiaire soient quaternisés ou avec un acide jusqu'à ce qu'au moins 98% des groupes amino tertiaire soient protonés.

12. Procédé selon la revendication 10 ou 11, **caractérisé en ce que** l'agent d'alkylation est le sulfate de diméthyle ou le chlorure de méthyle.

13. Procédé selon la revendication 10 ou 11, **caractérisé en ce que** l'acide est un acide carboxylique comprenant 1 à 3 atomes de carbone.

14. Composition d'adoucissement de textiles pouvant être obtenue selon un procédé selon l'une quelconque des revendications 10 à 13.

15. Adoucissant aqueux, contenant
2 à 25% en poids d'une composition d'adoucissement de textiles selon l'une quelconque des revendications 1 à 9 ou selon la revendication 14 et
70 à 98% en poids d'eau.

16. Utilisation d'une composition d'adoucissement de textiles selon l'une quelconque des revendications 1 à 9 ou selon la revendication 14 pour la préparation d'un adoucissant aqueux.
